(19) Europäisches Patentamt

European Patent Office

Office européen des brevets

(11) **EP 3 177 301 B1**

(12) **EUROPEAN PATENT SPECIFICATION**

(45) Date of publication and mention
of the grant of the patent:
**20.03.2019 Bulletin 2019/12**

(21) Application number: **15766194.3**

(22) Date of filing: **08.09.2015**

(51) Int Cl.:
*A61K 9/48* (2006.01)    *A61K 35/744* (2015.01)
*A61K 35/644* (2015.01)

(86) International application number:
**PCT/GB2015/052594**

(87) International publication number:
**WO 2016/038355 (17.03.2016 Gazette 2016/11)**

(54) **AN IMPROVED PROCESS FOR PRODUCING A SOFT GEL CAPSULE COMPRISING VIABLE PROBIOTIC BACTERIA AND A SOFT GEL CAPSULE COMPRISING VIABLE PROBIOTIC BACTERIA HAVING A LONG SHELF LIFE**

VERBESSERTES VERFAHREN ZUR HERSTELLUNG EINER WEICHGELKAPSEL MIT LEBENSFÄHIGEN PROBIOTISCHEN BAKTERIEN UND WEICHGELKAPSEL MIT LEBENSFÄHIGEN PROBIOTISCHEN BAKTERIEN MIT LANGER HALTBARKEIT

PROCÉDÉ AMÉLIORÉ POUR PRODUIRE UNE GÉLULE COMPRENANT DES BACTÉRIES PROBIOTIQUES VIABLES ET GÉLULE COMPRENANT DES BACTÉRIES PROBIOTIQUES VIABLES AYANT UNE DURÉE DE CONSERVATION LONGUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2014 GB 201415862**

(43) Date of publication of application:
**14.06.2017 Bulletin 2017/24**

(60) Divisional application:
**18194526.2 / 3 446 695**

(73) Proprietor: **Ayanda GmbH**
**16928 Pritzwalk (DE)**

(72) Inventors:
• **PHILIPP, Jessica**
**16928 Pritzwalk / OT Steffenshagen (DE)**
• **KURTMANN, Lone**
**2900 Hellerup (DK)**
• **WINNING, Mette**
**2860 Søborg (DK)**
• **KAPPELGAARD, Julie Mahler**
**3520 Farum (DK)**

(74) Representative: **Bakhtyari, Arash**
**adares**
**Patent- und Rechtsanwälte**
**Reininger & Partner**
**Tauentzienstrasse 7 b/c**
**10789 Berlin (DE)**

(56) References cited:
EP-A1- 0 634 167          WO-A2-2008/046625
US-A1- 2004 223 956       US-A1- 2012 107 395

**Description**

FIELD OF THE INVENTION

The present invention relates to the field of soft gel capsules comprising probiotic bacteria and to an improved process for producing a soft gel capsule comprising probiotic bacteria.

BACKGROUND OF THE INVENTION

For soft gel capsules comprising probiotic bacteria it is common practice to mention the number of probiotic bacteria at the time of manufacture or at the end of shelf life. Various attempts to produce soft gel capsules comprising viable probiotic bacteria having a long shelf life have been described in the prior art e.g. as summarized in WO 2012/021432 which is incorporated herein by reference.

WO 2012/021432 describes a process of manufacturing a soft gel capsule containing microencapsulated probiotic bacteria which is stable at room temperature for at least 24 months. The solution is to provide the probiotic bacteria with at least one coating comprising at least one vegetable lipid having a melting point of between 35°C and 75°C.

Tests of products presently commercially available showed that generally in the analyzed products the water activity level was relatively high, above 0.3. Often the water barrier properties of the applied packaging system were low e.g. an aluminum/PVC blister and that combined with no active desiccant material present after a certain time period has a detrimental effect of the number of viable bacteria in the products.

CA 2 675 892 describes packaging for soft gel capsules in a resealable container in the inside wall(s) of which an absorbent and at least one channel former is embedded, at least over part of the area. Examples of moisture-sensitive compounds which are preferably present in the soft gel capsules are vitamins and probiotic cultures. The examples of CA 2 675 892 show data for vitamins only. US 2012/107395 discloses soft gel capsules comprising a gelatin shell and a filling comprising probiotic and oil.

None of the above documents describe or point to the process for producing a soft gel capsule comprising viable probiotic bacteria nor to the soft gel capsules comprising viable probiotic bacteria of the present invention.

SUMMARY OF THE INVENTION

The present invention relates to a method for producing and storing a soft gel capsule comprising probiotic bacteria which process according to the claims has been improved by optimization of the production process to minimize the loss of viable cells during production. The method may be further improved by reducing the loss of viable cells during storage by carefully controlling the water activity during shelf storage to provide a soft gel capsule which comprises at least 1 E+09 viable bacteria after 24 months of storage at 25°C.

Probiotic bacteria are live microorganisms and this can be a challenge during production and formulation of final dosage forms. Probiotic bacteria are especially sensitive towards temperature, moisture content, and other ingredients in a formulation matrix.

Using a low fill temperature, and correspondingly using a gelatin with lower gelling and melting point than conventionally, ensures the survival of the probiotic bacteria during production. The wedge temperature (for filling and assisting the sealing), and the spreader box temperature (for forming the gelatin to a sheet suitable for entering the dierolls) must be adjusted accordingly. Mechanical stress must be avoided.

Further, the packaging of the soft gel capsules comprising probiotic bacteria should be optimized by carefully controlling the water activity during storage.

It is well known to the person of skill in the art that survival of probiotic bacteria is improved by a low water activity. However, a soft gel capsule will become brittle and leak if the water activity is too low. The optimal water activity is thus a delicate balance between survival of probiotic bacteria and intact soft gel capsules. It was found that the water activity should preferably be at the most 0.2 for storage at room temperature, such as 25°C.

DETAILED DISCLOSURE OF THE INVENTION

The present invention relates to a method for producing a soft gel capsule comprising uncoated probiotic bacteria, the method comprising mixing the uncoated probiotic bacteria with at least one oil to obtain a fill material at a temperature in the range of 5 to 15°C, encapsulating the fill material in a soft gel capsule made of a gelatin having a melting point in the range of 11 to 28°C; and drying the soft gel capsule in one or more steps at a temperature of at the most 25°C to a water activity of at the most 0.25.

Water activity ($a_w$) is defined as the partial vapour pressure of water in a composition at a specified temperature divided by the standard state partial vapour pressure of water at the same temperature. Water activity thus acts as a measure of the amount of free (i.e. unbound) water in a compsition. It may be calculated as:

$$a_W = p/p_0$$

where p is the partial vapour pressure of water in the composition and $p_0$ is the vapour pressure of pure water at the same temperature.

Alternatively, water activity may be calculated as:

$$a_W = l_w x_w$$

where $l_w$ is the activity coefficient of water and $x_w$ is the mole fraction of water.

The two calculations above which define $a_W$ are equivalent.

Water activity may be measured by methods known to those skilled in the art, for example by applying a Rotronic Hygrolab instrument.

A soft gel capsule is defined as a soft gelatin based shell surrounding a fill material. The term "fill material" refers to the entirety of materials encased within the shell. As such, the fill material can for example be a homogenous solution or a suspension.

The use of soft gel capsules in the delivery of dietary supplements or pharmaceutical products is widespread. The common soft gel capsule is mostly produced by the so-called Rotary Die Encapsulation process (invented by Robert Pauli Scherer), where two flat semi-solid ribbons of gelatin based shell material are produced from a liquid gelatin solution, the two ribbons are then brought together on a set of rotating dies (die rolls), which cut the gelatin ribbon, while sealing the two parts together. Simultaneously, precise addition of the correct amount of liquid fill material through the filling wedge expands the gelatin ribbon into the die pockets, before the final sealing of the soft gel capsule. After sealing, the still too soft gel capsule is dried, either in an in-line process or on trays in controlled atmosphere, in order to achieve the optimal hardness of the shell.

The fill material must be liquid at the production temperature used and also stable under the production conditions, but the flexibility in the composition of the fill material is large. The most common and easiest fill materials are purely oil based, such as fish oils or fat-soluble vitamins in an oil carrier. The ease of encapsulation and stability of the product will depend on any fill material/shell interactions, i.e. substances which are water soluble will interact with the water dissolved in the soft gel shell, altering the characteristics of the shell. Suspensions of solids in an oil carrier are also possible to incorporate into a soft gel, and in addition to the active ingredients, carriers/suspending agents to make a homogenous dispersion, antioxidants to prevent oxidation of the oil and excipients may be added if considered appropriate. The particle size of the solids in the suspensions should be low, and this may be achieved by milling the fill material prior to encapsulation.

A certain amount of solid, such as dried probiotic bacteria, may be included in the fill material but in order to keep the viscosity of the fill material at a level where it is possible to produce the soft gel, the amount of solid is limited to up to approximately 30% (w/w) of the fill material, preferably 10 to 25%, e.g. 15 to 20%. Increasing the amount of solid further will then mean increasing the amount of fill material, hence the capsule size. This can only be done to a certain extent otherwise the dimension of the soft gel capsule will be unrealistically large for a person to swallow. Generally, soft gel capsules have a weight in the range of 0.05 to 2.0 g, preferably 0.5 to 1.5 g, e.g. 0.7 to 1.0 g. Most often soft gel capsules are defined as a size (e.g. 10 oval, 12 oval etc. or oblong 4, 6 etc.).

Thus, in order to obtain a soft gel capsule comprising probiotic bacteria having a long shelf life a high degree of survival of viable bacteria has to be ensured during the production process and this high amount has to be maintained by carefully controlling the water activity of the soft gel capsules during shelf storage after the production and initial one or more drying step(s).

The present inventors realized that a substantial improvement of the production process to minimize the loss of probiotic bacteria through the production process by using a lower temperature of the fill material than conventionally used was necessary. The preparation and storage of the fill material is the most time-consuming unit operation, hence where the probiotic bacteria are most vulnerable. It was realized that when using a lower fill temperature than conventionally used also adjustments in the process conditions in the rest of the process in order to accommodate the lower fill material temperature are necessary.

Most importantly, the gelatin utilized must accommodate a low fill material temperature. The gelatin must have a lower gelling point and a lower melting point, than conventionally used so that the gelatin stays soft enough through the cold filling. This softness is important to make a proper seal by the final action of the dieroll; a bad seal will lead to leaking capsules.

The present inventors realized that fish gelatin generally has melting points below that of mammalian gelatins (Karim et Bhat, 2009). The different known fish gelatins generally have gelling points between 8 and 25°C, and melting points from 11-28 °C. The lower values belong to cold water fish gelatins that rarely have bloom strengths sufficient for soft gel production. The bloom strength generally used for soft gel production would typically be between 150-200 bloom (Podczeck et Jones, 2004), but based upon the experience of the present inventors the bloom strength could be between 100-300 bloom. Typical gelling points for porcine and bovine gelatins range from 20-25°C and typical melting points range from 28-31°C, with porcine gelatin exhibiting the highest gelling and melting points. Fish gelatins are generally derived from either cold water fish (for example cod, haddock, hake, pollock, cusk, sole, flounder, turbot, halibut, plaice, lump fish, redfish, pike, trout and salmon) or warm water fish (for example tilapia, shark or carp). These differ in their gelling and melting points, with cold water fish gelatins (CWFGs) often having gel points below 15°C, typically 4 to 12°C, and melting points below 22°C, typically 12 to 19°C, while warm water fish gelatins (WWFGs) often have gel points above 15°C, typically 18 to 24°C, and melting points in the range 22 to 32°C. For present purposes, cold water fish may be taken to mean any species of fish living predominantly in water of 18°C or below.

The present process uses a gelatin having a melting point in the range of 11 to 28°C. Preferably the melting point is in the range of 19 to 27°C, even more preferably in the range of 21 to 26°C, most preferably in the range of 23 to 25°C.

The present process preferably uses a fish gelatin having a bloom strength in the range of 100-300, such as at least 180, at least 190, preferably 200-285 or 200-250 bloom. However, a suitable bovine gelatin fulfilling the above parameters could also be suitable if it accommodates the parameters necessary to be used in the low temperature soft gel capsule production method according to the present invention.

Optionally the gelatin having a melting point in the range of 11 to 28°C may be a mixture of gelatins from different origins (e.g. a mixture of cold water fish gelatin and warm water fish gelatin, a mixture of fish gelatin and bovine gelatin, or a mixture of fish gelatin and porcine gelatin) provided that the overall gelatin mixture retains a melting point in the range of 11 to 28°C, preferably in the range of 19 to 27°C, even more preferably in the range of 21 to 26°C, most preferably in the range of 23 to 25°C, and/or a bloom strength in the range of 100-300, such as 200-285 bloom. The gelatin used, however, is preferably substantially free of mammalian gelatins, i.e. containing no more than 1%, preferably 0% wt relative to the total gelatin weight. Particularly preferably the gelatin is at least 90% wt, more preferably at least 95% wt, especially 100% wt, fish gelatin.

As noted above, cold water fish gelatins tend to have low or non-existent Bloom values and are therefore not always suitable for soft gel production (e.g. due to low mechanical strength and/or poor thermal stability). However, soft gel capsules composed of a significant amount of cold water fish gelatins can be manufactured using standard encapsulation equipment if an amount of warm water fish gelatin is also used. Suitable blends of cold water and warm water fish gelatins are described in WO 2009/095670. Thus, for example, in the method and capsule of the present invention the gelatin may be a blend of gelatins including at least 40 wt% cold water fish gelatin (relative to the total weight of gelatin), e.g. 20 to 80% wt, 35 to 70% wt, 40 to 65% wt, or at least 55% wt; or the gelatin may comprise cold water and warm water fish gelatin in a weight ratio of 99.9:0.1 to 35:65, especially 65:35 to 40:60; provided that the overall gelatin mixture retains a melting point in the range of 11 to 28°C and/or a bloom strength in the range of 100-300.

Optionally the gelatin employed in the present invention may further comprise a carrageenan (e.g. kappa-carrageenan), for example at a content of 0.01 to 2% wt of the gelatin on a dry solids basis, especially 0.05 to 1% wt, particularly 0.06 to 0.5% wt. To improve gelling, a physiologically tolerable metal salt (e.g. a potassium salt such as potassium chloride for kappa-carrageenan), may also be included. Typically this will be at a concentration in the gelatin of up to 50mM, especially 10 to 30mM.

Gelatins suitable for use in the capsules and methods of the invention may of course be defined in terms both of bloom strength and melting point. Thus gelatins having any combination of the above bloom strengths and melting points are contemplated, for example a gelatin having a melting point in the range 11 to 28°C and a bloom strength of 100 to 300 bloom.

The soft gel capsule shell may, in addition to gelatin, contain additional ingredients such as plasticizer(s), water, colorant(s), flavor(s), opacifier(s), and/or preservative(s), which are all known ingredients to the person skilled in the art (see also Stanley, J.P., "Part Two. Soft Gelatin Capsules", in The Theory and Practice of Industrial Pharmacy, Lachmann, L., et al., eds. Lea & Febiger, Philadelphia, PA, pp. 398-412 (1986).

Examples of plasticizers include glycerol, sorbitol and mixtures thereof. Where present, the plasticizer may be present in an amount of up to 35% wt, e.g. 5 to 35% wt or 15 to 30% wt of the gelatin.

A probiotic bacterium is defined as a live microorganism which when administrated in adequate amounts may confer a health benefit to the host (FAO/WHO 2001).

Under conditions of lack of nutrition certain bacteria such as *Bacilli* and *Clostridia* are able to form endospores, a dormant, tough, non-reproductive structure. Endospores can survive without nutrients. They are resistant to ultraviolet radiation, desiccation, high temperature, extreme freezing and chemical disinfectant. Some probiotic products contain *Bacilli.* As evident from the above, the method of the present invention is, however, not necessary for spore forming bacteria to survive the encapsulation process. In a preferred embodiment, therefore, the bacteria employed in the soft gel capsules of the invention are non-spore-forming bacteria.

The fill material comprises at least one species of a dried uncoated non-spore forming probiotic bacterium. Bacteria may be dried according to methods known in the art. Examples of such probiotic bacteria are *Lactococcus, Lactobacillus, Pediococcus,* and *Streptococcus* and more preferably at least one species selected from the group consisting of *Bifidobacterium* spp., *Bifidobacterium breve, Bifidobacterium animalis, Bifidobacterium lactis, Bifidobacterium longum, Bifidobacterium bifidum, Lactococcus lactis, Lactococcus cremoris, Lactobacillus acidophilus, Lactobacillus casei, Lactobacillus kefir, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sake, Lactobacillus reuteri, Lactobacillus lactis, Lactobacillus delbreuckii, Lactobacillus plantarum, Lactobacillus johnsonii* and *Streptococcus thermophilus.*

Particularly preferred strains are *Bifidobacterium animalis* subsp *lactis,* e.g. the strains deposited as DSM 15954 (marketed by Chr. Hansen A/S, Denmark, as BB-12®); ATCC 27536, and DSM 10140, respectively; *Lactobacillus acidophilus,* e.g. the strain deposited as DSM 13241 (marketed by Chr. Hansen A/S, Denmark, as LA-5®), *Lactobacillus rhamnosus,* e.g. the strain deposited as ATCC 53103 (marketed by Chr. Hansen A/S, Denmark, as LGG®), *Lactobacillus paracasei* subsp. *paracasei,* e.g. the strains deposited as ATCC 55544, (marketed by Chr. Hansen A/S, Denmark, as L. casei 431®) and CCTCC M204012, respectively, *Lactobacillus reuteri,* e.g. the strain deposited as ATCC 55845 (marketed by Chr. Hansen A/S, Denmark, as RC-14®), *Lactobacillus rhamnosus,* e.g. the strain deposited as ATCC 55826 (marketed by Chr. Hansen A/S, Denmark, as GR-1®), *Lactobacillus paracasei,* e.g. the strain deposited as LMG-P-17806 (marketed by Chr. Hansen A/S, Denmark, as F19®), *Streptococcus thermophilus,* e.g. the strain deposited as DSM 15957 (marketed by Chr. Hansen A/S, Denmark, as TH-4®), and *Lactobacillus fermentum,* e.g. the strain deposited as NM02/31074 (marketed by Chr. Hansen A/S, Denmark, as PCC®).

Combinations of several species or strains of probiotic bacteria can be used, i.e. 2, 3, 4, 5, 6, 7, 8, 9, 10, 11, 12, 13, 14, 15, 16, 17, 18, 19, 20, 21, 22, 23, 24, 25 or even more of the above listed species and strains. In presently preferred embodiments, only one, two, three, four or five different strains are present in the soft gel capsule according to the invention.

By the term "viable" is meant that the cell is alive and capable of forming a colony in a petri dish during pour plating or spread plating. The number of viable probiotic bacteria is determined as the number of colony forming units (CFU) by pour plate or spread plate methods with incubation under conditions suitable for growth of the probiotic strain(s). By this method cells capable of growing and forming colonies will be counted. When a number is given in the present specification and claims, it should be understood as CFU/soft gel capsule unless the context indicates otherwise.

The terminology used to describe micro-particulate formulations can sometimes be inconsistent and confusing to readers unfamiliar with the field. The term "microparticle" as used herein refers to a particle with a diameter of 1-1000 μm, irrespective of the precise interior or exterior structure and the subcategory of "micro-capsules" applies to microparticles which have a core surrounded by a material which is distinctly different from that of the core. The core may be solid, liquid, or even gas.

Microencapsulated materials have a core active ingredient, surrounded by a material that is distinctly different. A substance may be microencapsulated for a number of reasons. Examples may include protection of reactive material from their environment, safe and convenient handling of the materials which are otherwise toxic or noxious, taste masking, means for controlled or modified release properties, means of handling liquids as solids, preparation of free flow powders and in modification of physical properties of a drug.

WO2012/021432 describes microencapsulated probiotic bacteria and defines the term "microencapsulated" as coated with a composition. Consistent herewith, the term "uncoated" used in the present specification and claims means that the probiotic bacteria are not microencapsulated or coated.

A coating step may well lead to a cell loss during the coating process and a different release profile than natural, uncoated cells, and will also lead to a lower concentration of probiotic bacteria through the addition of coating material to the formulation.

As evident when comparing the results of the examples of the present invention with the results of the examples in WO2012/021432, the present invention provides an improved survival of the probiotic bacteria during long term storage at room temperature compared to the results described in WO2012/021432 even without using coating of the probiotic bacteria. The bacteria employed in the present invention are therefore uncoated, i.e. non-microencapsulated.

An excipient may be added to the probiotic bacteria, e.g. in order to obtain a dry blend with a standardized concentration of probiotic bacteria. Presently preferred embodiments have end concentration of 40-100% (w/w) in the blend of one or more active ingredients such as probiotic bacteria, such as 50%, 60%, 70%, 80% or 90%, the remaining part being excipient(s) such as maltodextrin. A concentration of 100% (i.e. dried bacteria only) is presently preferred in order to have a high concentration of bacteria and to reduce the amount of solid to be added to the oil - adding too much solid makes the suspension too viscous.

In order to make a suspension of the dried bacteria for the fill material, a suspending agent, for example a hydrogenated/partly hydrogenated vegetable oil, mono-glyceride or mixture thereof, lecithin, a wax or silicon dioxide may be added in a range of 1-10% (w/w) of the total fill material, such as 1.5-7.5%, e.g., 1.5-5% (w/w). The use of a suspending agent aids in the preparation of a homogeneous fill material by preventing sedimentation of the probiotic bacteria even though the bacteria are added in dry powder form.

Essentially all edible oils can be suitable for the present invention. The oil may be considered as an active ingredient or a carrier oil only, such as MCT (medium chain triglyderide), soya bean oil, or sunflower oil. Particularly preferred oils for use according to the present invention are oils containing omega 3 fatty acids, such as docosahexaenoic acid (DHA), eicosapentaenoic acid (EPA), fish oil, and krill oil, and combinations of EPA, DHA and other omega 3 fatty acids, and vegetable oils providing e.g. DHA, EPA, alpha-linolenic acid (ALA), or gamma linolenic acid (GLA), such as rapeseed oil, borrage oil/evening primrose oil, linseed oil, perilla oil, or garlic oil.

Sources of DHA, EPA and ALA include, but are not limited to, fish oils, yeasts, algae or other microorganisms or monocellular sources and vegetable oils, primarily flaxseed, soy, and rapeseed oil. Sources of GLA include, but are not limited to, evening primrose oil/borage oil.

Oils suitable for use according to the present invention also includes any modified oil from vegetable, marine or mammal origin, such as, but not limited to hydrogenated oils, diacylglycerols, monoacylglycerols, medium chain triglycerides (MCT) and combinations thereof.

At least one oil, such as one, two, three, four or more of the above listed oils, may be used in the method of the present invention.

As noted above, the method of the present invention involves mixing the uncoated probiotic bacteria with at least one oil to obtain a fill material at a temperature in the range of 5 to 15 °C. Therefore, the oil (or oil mixture, where two or more oils are used) should be liquid at temperatures in the range 5 to 15 °C. This means that the oil (or oil mixture) should have a melting point of not more than 15 °C, e.g. a melting point in the range 5 to 15 °C or preferably a melting point below 5 °C.

In addition to the probiotic bacteria, one or more other active ingredients, for example one, two, three, four or more active ingredients selected from the group consisting of vitamins such as vitamin A, D, E, K2, C, B2, B6, B12, biotin, niacin, folic acid; minerals such as zinc, selenium, chromium, copper, calcium, chloride; vegetable oils such as rapeseed oil, borrage oil/evening primrose oil, linseed oil, perilla oil, garlic oil, and vegetable extracts such as cranberry extract/juice, royal jelly could be included in soft gel capsules.

Presently preferred compositions include *Bifidobacterium animalis* subsp *lactis* as the only active ingredient, such as *Bifidobacterium animalis* subsp *lactis* in MCT; *Bifidobacterium animalis* subsp *lactis* and at least one omega-3 oil, such as *Bifidobacterium animalis* subsp *lactis* and ALA, *Bifidobacterium animalis* subsp *lactis* and DHA, and *Bifidobacterium animalis* subsp *lactis,* DHA and EPA; as well as combinations with other active ingredients e.g. *Bifidobacterium animalis* subsp *lactis,* DHA, vitamin B6, folic acid, and Vitamin D3.

The mixing of the fill material and the encapsulation of the fill material into the soft gel capsule should take place in a low humidity environment. In the context of the present invention, the phrase "low humidity environment" refers to an environment with a relative humidity (RH) of less than 50%. Preferably, it refers to an environment with a relative humidity of less than 45%. More preferably, the relative humidity is in the range of 10%-40%, more preferably in the range of 20-35%. The low humidity may be reached by any method known the person skilled in the art for reducing the relative humidity, and includes the use of dry compressed air, cooling, air conditioning, and purging with an inert gas e.g. nitrogen or argon. Purging with an inert gas also reduces oxidative stress both on the bacteria cells and other components of the fill material.

In order to ensure good content uniformity in the soft gel capsules it is important to keep the bacteria evenly suspended in the oil. Gentle agitation and high viscosity, obtained by e.g. lowering the temperature, can assist in making an even suspension of the bacteria. By "gentle" is meant agitation which is sufficient to assist the formation of a homogeneous suspension without giving rise to mechanical forces which cause significant disruption of bacterial cell walls. Suitable methods for gentle agitation/mixing include, but are not limited to, stirring or circulation pumping. Other methods will be identifiable by those skilled in the art.

Table 1 summarizes the differences between the conditions used in the method of the present invention and the conditions generally used in this field.

Table 1

| Production parameters for production of probiotic soft gel capsules | | |
| --- | --- | --- |
| Parameter | Standard values | Probiotic production values |
| Spreader box temp. | 50 - 68°C | 40 - 68°C |
| Wedge temperature | 28 - 48°C | 28 - 36°C |

(continued)

| Production parameters for production of probiotic soft gel capsules | | |
|---|---|---|
| Parameter | Standard values | Probiotic production values |
| Room temperature | 18 - 22°C | Max 20°C |
| Fill temperature | Room or above | 8 - 15°C |

Reduction of the fill material temperature necessitates the use of a lower melting gelatin and changes in the whole production chain. Most importantly, the wedge temperature (involved in the precise filling and subsequent sealing of the capsule) must be reduced in order to have a good seal of the capsule (not re-melting the gelatin). The spreader box will ensure that the liquid gelatin mass is formed into a thin film before the encapsulation process. The temperature in the spreader box will not heat the fill material, and the upper temperature limit may be as high as for normal production, although preferably lower.

As evident from the table the major difference from the conventional process is the lower fill temperature. Example 1 clearly demonstrates that it is important to lower the temperature of fill material from the conventionally used room temperature. According to the method of the present invention, the fill temperature should be 5-15°C, preferably 6 to 14°C, most preferably 7 to 13°C. 8 to 12°C, such as 9 to 11°C may also be feasible.

The temperature depends on the gelatin chosen. The use of a lower melting fish gelatin ensures that the sealing process of the capsule is still effective; with the colder fill material and wedge, the gelatin must still be soft enough to give a solid seal after the filling process. It is contemplated that the spreader box temperature may be as low as 40°C to adjust to lower room and fill temperatures, preferably in the range of 45-55°C. In the examples the lowest spreader box temperature used was 51 °C.

The particle size of any solid component of the fill material i.e. the dried probiotic bacteria and possible excipient(s) such as maltodextrin, is important to ensure homogeneity of the product, and enable the actual physical process. The average particle diameter size of the component comprising the probiotic bacteria should be less than 400 microns, such as about 150 to 250 microns, and preferably less than 200 microns. The particle size for smooth large scale production must be optimized, and can be obtained by careful particle size reduction by milling or sieving.

Due to the fact that the probiotic bacteria are delicate living microorganisms, it is, however, important to minimize stress to the probiotic bacteria/energy input. Thus, in order to maintain the viable count of bacteria procedures such as milling of the solid components of the fill material should be avoided to the extent possible.

The soft gel capsules according to the invention are to be firm, but not brittle or fragile The quality of the capsule shell after drying is most commonly measured as Newton (hardness of shell), where a value of 9-11 N designates a shell hardness that will not cause leaking capsules. Hardness may be determined by methods known in the art, for example by use of a hardness tester such as the digi test II Gelomat Härtemessgerät (manufactured by Bareiss Prüfgerätebau GmbH) and other similar devices.

At room temperature (25°C/60% relative humidity (RH)), soft gel capsules have a water content of about 4 to 10% by weight. If the water content is significantly below this, for example due to excessively dry storage or drying, this results in embrittlement of the capsule shell, with the consequences of increased fragility of the capsule wall and the formation of cracks. In an embodiment the soft gel capsules therefore have a water content of at least 4 wt%.

As will be known by the person skilled in the art, the water content refers to the total water. The total water content of the soft gel capsule (free water + bound water i.e. water bound in cells and gelatin) will mostly be found in the shell, since the hydrophobic capsule fill material will not absorb water. As stated above, the total water content in the capsule can be in the range of 4-10%, but the total percentage is not relevant for the survival of the bacteria. The water which is tightly bound by e.g. hydration of the gelatin in the shell cannot be utilized or be harmful to the bacteria, and it is only the water which is free to utilize which needs to be measured (the water activity, $a_w$). Water activity is by definition the free or non-chemically bound water in a product and can be measured on the whole soft gel capsule by applying a Rotronic Hygrolab instrument.

The probiotic bacteria are dormant, and to keep them in this condition, a water activity of not more than 0.2 is preferable. Normally, a soft gel capsule stored under standard soft gel conditions would create an environment with a water activity ($a_w$) which is too high for the probiotic bacteria to stay dormant. Thus, a careful control of the drying of the soft gel capsule comprising the probiotic bacteria, and a careful control over the atmospheric water that can re-enter the soft gel is necessary.

During the drying process the water content, hence the water activity, reach an equilibrium through a process where desiccant, if present, removes water from the shell, and any water in the fill material is taken up by the shell. The equilibrium between the shell, fill material and desiccant ensures the correct low water activity, without risking that the shell becomes too brittle.

According to the method of the present invention, the soft gel capsules are dried in one or more steps at a temperature of at the most 25°C to a water activity of at the most 0.25. Any drying method known to a person of skill in the art may be used for drying the soft gel capsules which comprise the bacteria. In a preferred embodiment of the invention, the capsules are primarily dried by tumble drying at for example about 18 to 23°C for from about 10 to about 40 minutes, followed by tray drying at about 19 to 22°C at a humidity of 15 to 22% for at least about 1 or 2 days to about 14 days until a shell hardness of 9-11 N is obtained. Alternatively to the above method, in-line drying tunnels can be used.

After the initial drying process, the dried soft gel capsules are sorted and capsules not being properly filled, having air bubbles or odd sizes are discarded. The quality checked soft gel capsules can then be packaged in the final packaging with a sufficient amount of desiccant as will be known to a person of skill in the art.

It is, however, presently preferred to add a further drying step where the soft gel capsules are kept as a bulk in an appropriate packing material with a sufficient amount of desiccant so as to obtain a further, slow reduction in water content with a target water activity ($a_w$) of 0.2 measured on the whole soft gel capsule. The capsules are kept at a temperature of at the most 25°C, preferably at a low temperature such as 2-8°C, in this phase, so as to minimize the loss of bacteria before packing in the final packaging.

As evident from the above the desired low water activity does not need to be reached before packing into final packaging, but the correct water activity before the packing into the final packaging will prolong the life of the desiccant in the final packaging and therefore also the shelf life of the soft gel capsules. Preferably the water activity should be ≤0.25 before final packaging.

The time needed to reach this low $a_W$ can be in a range from days, weeks to months, e.g. 3 months, but as long as the capsules are kept at a low temperature, the stability is not compromised.

The most suitable packing material for this bulk packing is aluminum foil based packing materials. Other packing materials such as HDPE polymers, or other high water barrier polymers can be used, but then an increased amount of desiccant is needed to control the water activity. Suitable desiccants include any suitable desiccant such as silica, silica based desiccants, molecular sieves, and zeolites.

In a presently preferred embodiment of the invention, the soft gel capsules are dried after the final repackaging (i.e. during the shelf storage) to a water activity ($a_w$) of at the most 0.2, such as 0.08, 0.09, 0.10, 0.11, 0.12, 0.13, 0.14, 0.15, 0.16, 0.17, 0.18, 0.19, 0.20, 0.21, 0.22, or 0.23.

Capsules obtained or obtainable by the methods herein described form a further aspect of the invention.

In another aspect, the present invention provides a soft gel capsule comprising a capsule shell and a fill material, said capsule shell comprising at least one gelatin having a melting point in the range 11 to 28°C and/or a Bloom value of 100 to 300, and said fill material comprising at least one strain of uncoated, non-spore-forming probiotic bacteria dispersed in an oil, wherein said soft gel capsule a water activity of at most 0.25.

Containers made from polymers which comprise absorbents and channel formers and which are presently preferred for the final packaging are described, for example, in WO 97/32663, WO 03/086900, WO2008/086852, EP 1000873, and EP 1421991. They are commercially available, for example, from Capitol Specialty Plastics Inc., 2039 McMillan Street Auburn, Alabama, USA, under the trade name Activ-Vial or from Sud Chemie, Ostenrieder Str. 15, 85368 Moosburg, Germany, under the trade name 2 AP Multipolymer.

The present invention provides a soft gel capsule comprising dried, uncoated, non-spore-forming probiotic bacteria which soft gel capsule comprises at least 2 E+09 viable bacteria after 12 months of storage at 25°C, more preferably at least 3 E+09 viable bacteria, most preferably at least 4 E+09 viable bacteria, even more preferably at least 5 E+09 viable bacteria.

Within the scope of the invention are a soft gel capsule comprising dried, uncoated, non-spore-forming probiotic bacteria which soft gel capsule comprises at least 1 E+09 viable bacteria after 24 months of shelf storage at 25°C, preferably at least 2 E+09 viable bacteria, more preferably at least 3 E+09 viable bacteria, even more preferably at least 4 E+09 viable bacteria, most preferably at least 5 E+09 viable bacteria.

Further, the invention provides a soft gel capsule comprising dried, uncoated, non-spore-forming probiotic bacteria which soft gel capsule comprises at least 4 CFU/g after 12 months of shelf storage at 25°C, preferably at least 5 CFU/g, more preferably at least 6 CFU/g, even more preferably at least 7 CFU/g, most preferably at least 8 CFU/g.

Even further the invention provides a soft gel capsule comprising dried, uncoated, non-spore-forming probiotic bacteria which soft gel capsule comprises at least 2 CFU/g after 24 months of shelf storage at 25°C, preferably at least 3 CFU/g, more preferably at least 4 CFU/g, even more preferably at least 5 CFU/g, most preferably at least 6 CFU/g.

As evident from the results provided in the examples, the soft gel capsules prepared by the method of the present invention are very stable. The results show a CFU value per soft gel capsule of 6.8 E+09 after 6 months of storage, 4.0 E+09 after 9 months of storage and 5.3 E+09 after 12 months at 25°C in plastic vials with desiccant lining. Based on the CFU values after 12 months of storage it is possible to extrapolate the number of viable probiotic bacteria likely to be present after 24 months of storage at 25°C to be at least 3 E+09.

An alternative way of measuring the storage stability of soft gel capsules is to calculate the log loss of probiotic bacteria during the shelf storage. The method of the present invention provides for a log loss during the shelf storage which is not more than 1 after 12 months of storage at 25°C, preferably not more than 0.75, more preferably not more than 0.50, even more preferably not more than 0.33, most preferably not more than 0.25.

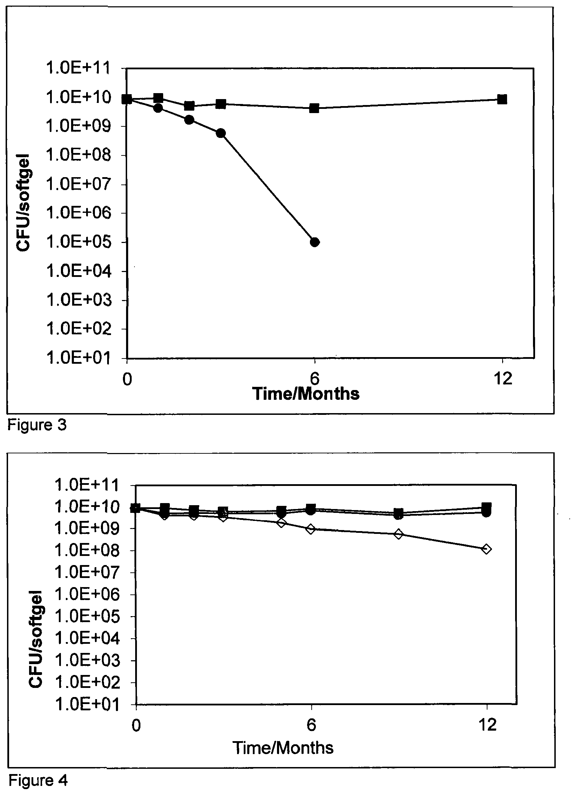

The examples provide that the log loss for soft gel capsules stored in active plastic vials at 25°C after 6 months of storage can be calculated to 0.12 and the log loss after 12 months to 0.23. As can be seen from Figure 4 there is over time some variation of the exact numbers most likely due to variation in the distribution of the probiotic bacteria between the individual soft gel capsules and due to the analytical variation on the CFU method but the overall result shows that the soft gel capsules according to the invention maintain a remarkably low log loss.

When the soft gel capsules with the probiotic bacteria are kept at 2 to 8°C, the loss of cells is even lower than at 25°C as evident from Figures 3 and 4. Figure 3 also shows that the bulk storage conditions can be optimized by adding further desiccant to control the water activity level already on bulk level.

As appears from Figure 4 the survival of the probiotic bacteria is much higher when the soft gel capsules containing probiotic bacteria are stored at 25°C compared to the results when the soft gel capsules were stored at 30°C. It is surprising that the 5°C change in storage temperature from 25°C to 30°C can provide such a significant difference in survival of the bacteria. Preferred storage conditions according to the invention are storage at room temperature such as at a temperature of from about 20 to about 25°C, such as about 22 to 25°C, preferably about 25°C, i.e. 25°C $\pm$2°C.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

FIGURES

Figure 1 shows CFU values of soft gel capsules produced in trial 2 during storage at 25°C/60%RH for up to 18 months. The soft gel capsules are packed in three different packaging systems. (▪)Aluminum foil+ silicate bag, oblister card, (•) box+silicate bag.

Figure 2 shows CFU values of soft gel capsules produced in trial 2 during storage at 8-12°C for up to 18 months. The soft gel capsules are packed in three different packaging systems. (•)Aluminum foil+ silicate bag, oblister card, (•) box+silicate bag.

Figure 3 shows CFU values of soft gel capsules produced in trial 4 packed in simulated bulk packaging systems and stored at 5°C and 25°C/60%RH for up to 12 months. (▪) 5°C, and (•) 25°C/60%RH.

Figure 4 shows CFU values of soft gel capsules produced in trial 4 packed in plastic vials with desiccant lining during storage at 5°C, 25°C/60%RH and 30°C/75%RH for up to 12 months. (▪)5°C, (•)25°C/60%RH , and ◊ 30°C/75%RH.

Figure 5 shows shows CFU values of soft gel capsules produced in trial 4 packed in simulated bulk packaging systems and stored at 5°C and 25°C/60%RH for up to 24 months. (▪) 5°C, and (•) 25°C/60%RH.

Figure 6 shows CFU values of soft gel capsules produced in trial 4 packed in plastic vials with desiccant lining during storage at 5°C, 25°C/60%RH and 30°C/75%RH for up to 24 months. (▪)5°C, (•)25°C/60%RH, and ◊ 30°C/75%RH.

EXAMPLES

General:

All trial batches have a batch size of 65,000 capsules.

Fill material:

The oil and oil soluble components are charged by vacuum to a stainless steel mixing vessel. The components are mixed at 12 °C, and added silicon dioxide. The mixture is further mixed and homogenized until the silicon dioxide is dispersed. *Bifidobacterium animalis* subsp. *lactis* is charged by vacuum and the complete mixture is mixed (without homogenization). The finished fill material is then discharged through a sieve with a sieve opening between 0.7 and 1.2 mm to a pre-cooled (12 °C) transfer tank. The transfer tank is then connected to an encapsulating machine.

Gelatin solution

The batch size of the gelatin solution is 100 kg. Gelatin, glycerol and water are weighed into stainless steel production vessel equipped for heating and stirring. The mixture is heated, stirred and evacuated until a stable viscosity is achieved. The gelatin is then filtered (150 microns) into a preheated stainless steel container. Coloring agents are then added and slowly mixed until homogenous. The gelatin is held at 50-68°C during encapsulation.

Drying

The first drying is performed in rotary tumble dryers at 19.0 to 20.3°C , where the lubricant of the gelatin ribbons is removed by cleaning towels in the dryers. Final drying is done by spreading the capsules on trays in a single layer, and placing the trays in air-conditioned cabinets at controlled humidity of 14-23% RH and temperature of 21-24°C. Drying is terminated when a shell hardness of 9-11 N is reached. The typical drying time is 31-130 h, with an average of 100 hours.

Inspection and packaging

The soft gel capsules are inspected and sorted to remove deformed capsules, capsules not properly sealed and other errors and packaged in bulk packages of 3500 soft gel capsules containing probiotic bacteria with 30 g of desiccant in an aluminum bag and heat sealed.
In Table 2 the composition of the batches produced in five different trials are shown.

Table 2

| Composition of five different batches produced | | | | | |
|---|---|---|---|---|---|
| Trial No | 1 | 2 | 3 | 4 | 5 |
| Ingredients in mg per capsule, 10 oval | | | | | |
| *Bifidobacterium animalis* subsp. *lactis* | 21.875 | 120.000 | 50.000 | 50.000 | 50.000 |
| Particle size, average, $\mu$m by microscopy | <730 | <166 | | | |
| Particle size, average, $\mu$m by laser scattering | | | 139 | 139 | 223 (unfiltered) 115 (filtered) |
| D90 (90% of batch), mm | | | 291 | 291 | 465 (unfiltered) 231 (filtered) |
| D-a-Tocopherol 1000 | 9.000 | 9.000 | 9.000 | 9.000 | 9.000 |
| Medium Chain Triglycerides | | | 500 | | |
| Total fish oil | 550.000 | 500.000 | | 500.000 | 500.000 |
| Silicon dioxide | 25.125 | 25.000 | 9.300 | 31.000 | 31.000 |
| Fish gelatin | 139.20 | 139.20 | 140.00 | 140.00 | 140.00 |
| Glycerine 99.5% | 63.86 | 63.86 | 67.49 | 69.96 | 67.49 |
| Water | 16.94 | 16.94 | 5.75 | 4.82 | 5.75 |
| Lemon Oil | | | 3.50 | 3.50 | 3.50 |
| Colouring agent * | | 4.36 | 3.26 | 1.72 | 3.26 |
| Total weight of fill | 606.0 | 654.0 | 568.3 | 590.0 | 590.0 |

(continued)

| Composition of five different batches produced | | | | | |
|---|---|---|---|---|---|
| Trial No | 1 | 2 | 3 | 4 | 5 |
| Total weight of capsule | 826.0 | 878.0 | 788.3 | 810.0 | 810.0 |
| *Iron Oxide, Titanium oxide, riboflavin | | | | | |

During the production of the two first trial batches, the temperature was closely monitored throughout the encapsulation process in order to identify where reduction of number of CFU took place.

The soft gels produced in trial 1 showed significant loss of viable cells through the production process. Changing the processing conditions and repeating the trial gave a significantly more stable production process. The results are shown below in Table 3:

Table 3

| Comparison of the temperatures and CFU values during the first two trials | | | | | |
|---|---|---|---|---|---|
| Trial no. | | 1 | | 2 | |
| Sample | Sampling point | CFU/capsule | Fill Temp./°C | CFU/capsule | Fill Temp./°C |
| Mixing vessel | Upper part | 1.39 E+09 | 20 | 1.90 E+10 | 7.5 |
| Mixing vessel | Lower part | 1.64 E+09 | 20 | 7.20 E+09 | 7.5 |
| Capsules | Beginning of encapsulation | 1.84 E+04 | 20 | 1.31 E+08 | 7.5 |
| Capsules | Middle of encapsulation | 5.10 E+05 | 20 | 1.24 E+08 | 7.5 |
| Capsules | End of encapsulation | 1.70 E+05 | 20 | 4.97 E+07 | 7.5 |
| Capsules | Rotary dryer, start | 2.12 E+05 | 20 | 2.62 E+08 | 20 |
| Capsules | Rotary dryer, middle | 1.64 E+05 | 20 | 3.00 E+08 | 20 |
| Capsules | Rotary dryer, end | 5.64 E+05 | 20 | 4.91 E+08 | 20 |
| Capsules | Tray drying, start | 4.73 E+05 | 19 | 2.94 E+09 | 20 |
| Capsules | Tray drying, middle | 1.03 E+08 | 19 | 2.62 E+09 | 20 |
| Capsules | Tray drying, end | 7.27 E+04 | 19 | 2.49 E+09 | 20 |

The impact of the reduction in fill temperature from 20°C to 7.5°C is evident, the survival of the probiotic bacteria is significantly better.

Looking more into the details of the results, Table 3 shows a drastic reduction of CFU/soft gel capsule during the encapsulation in trial 1 and that even at the low fill temperature in trial 2, the cell count is reduced through the encapsulation process. During drying the CFU/capsule is increasing, the reason being that during drying water is lost, so that the total concentration of bacteria increases (most clearly for trial 2).

This temperature was used for the subsequent trials except for some variations in the temperatures for the fill material (12-14°C). In the subsequent trials only a minimal production loss was found. As an example, in trial number 4 probiotic bacteria were added corresponding to a calculated amount of 3.0 E+10 CFU/soft gel capsule if all the cells would be available after the soft gel capsule production. The CFU value found when the stability study was started was 9E+09 CFU/soft gel capsule. These two values confirm the minimal loss of probiotic bacteria observed when manufacturing soft gel capsules comprising probiotic bacteria applying the improved process.

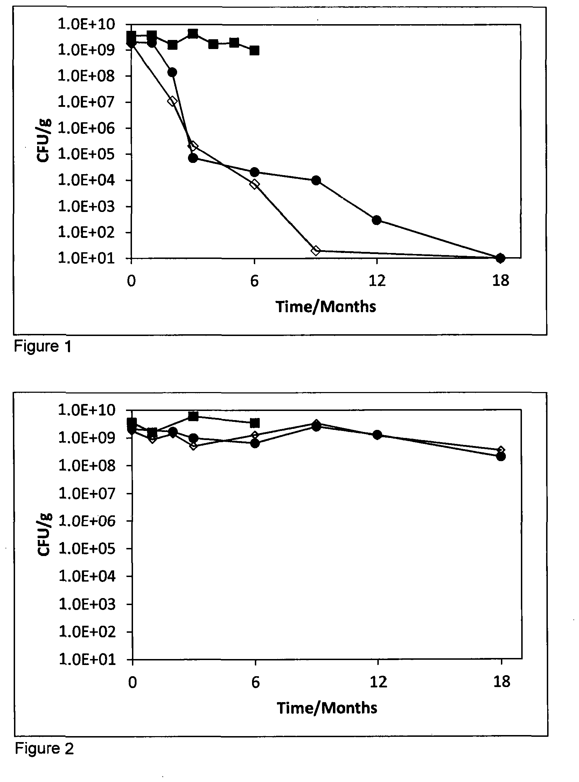

The batch of trial 2 was distributed into different packaging systems having different amounts of drying media availability and stability studies were initiated at different temperatures. The packaging systems were a) an aluminum foil bag containing 100 soft gel capsules and a 1g silica bag, b) a blister card (PVC/PVDC foil, 10 soft gel capsules per blister card and c) an aluminum bottle with PE plug and PP cap with ALU/PE liner containing 60 soft gel capsules and a 0.5g silica bag. The samples were stored at 8-12°C and at 25°C/65%RH for up to 18 months. The results are provided in Figures 1 and 2.

The results in Figure 1 show the importance of having an active desiccant present in the packaging system for the probiotic soft gel capsules.

The results in Figure 2 demonstrates that if the storage temperature is low - in the range of 8-12°C - the effect of desiccant material present in the packaging is less important for the CFU values of the soft gel capsules comprising probiotic bacteria.

The soft gel capsules from trial 4 have been included in various stability studies. In Figures 3 and 4 and Figures 5 and 6 some of the results are presented.

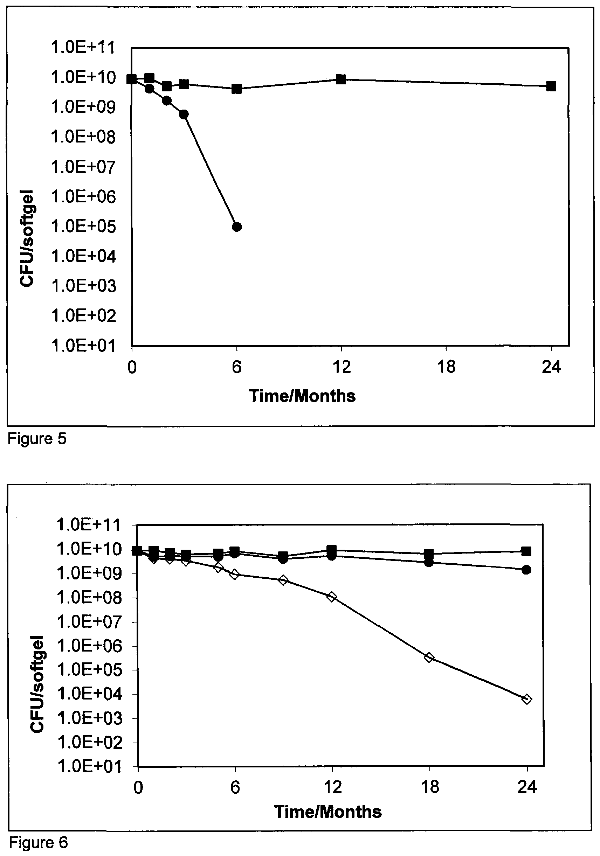

The stability as bulk soft gel capsules has been studied. In Figures 3 and 5 the total cell count during storage at 5°C and 25°C in simulated bulk packaging systems are shown (aluminum foil pouch each containing 30 soft gel capsules and 3.2 g desiccant material). The balance between the number of soft gel capsules and amount of desiccant material is not appropriate in order to make the soft gel capsules stable at 25°C. As evident from the figure the number of viable cells starts to decrease already after 1 month of storage at 25°C. In Table 4 the water activities measured during bulk storage are presented.

Table 4.

| Water activities during bulk storage | | |
|---|---|---|
| Months | Water activity | |
| | 5°C | 25°C/ 60%RH |
| 0 | 0.24 | 0.24 |
| 1 | 0.21 | 0.22 |
| 2 | 0.23 | 0.22 |
| 3 | 0.20 | 0.23 |
| 6 | 0.18 | 0.20 |
| 12 | 0.22 | - |
| 24 | 0.20 | - |

The bulk and final packaging data demonstrate the importance of controlling the water activity of the soft gel capsules during storage in order to make them as stable as possible.

The stability of the soft gel capsules in final packaging has been studied. 30 probiotic soft gel capsules were packed in plastic vials Active Vial, M-3009-336, Capitol Specialty
Plastics Inc., 2039 McMillan Street Auburn, Alabama, USA with a desiccant lining (4.3 g molecular sieve).

The storage conditions were 5°C, 25°C/60%RH and 30°C/75%RH. The stability study is on-going and the CFU values after 12 months of storage are available as shown in Figure 4. The CFU values after 24 months of storage are also available as shown in Figure 6. It is evident that the samples stored at 5°C or 25°C/60%RH have a low log loss whereas the log loss for samples stored at 30°C/75%RH is higher.

In Table 5 the corresponding water activities measured during storage are presented. The water activity decreases during the first month of storage due to the desiccant lining in the plastic vial and this has a stabilizing effect on the CFU value.

Table 5

| Water activities during storage in plastic vials | | | | |
|---|---|---|---|---|
| Time/Months | Water activity | | | Appearance |
| | 5°C | 25°C/ 60%RH | 30°C/ 75%RH | 5°C, 25°C/ 60%RH, and 30°C/ 75%RH |
| 0 | 0.24 | 0.24 | 0.24 | |
| 1 | 0.20 | 0.14 | 0.12 | |
| 2 | 0.22 | 0.16 | 0.13 | |
| 3 | 0.18 | 0.14 | 0.09 | |
| 5 | 0.14 | 0.12 | 0.12 | At all sampling at the three storage conditions the soft gel capsule are red-brown, and no leakages are observed |
| 6 | 0.12 | 0.06 | 0.08 | |
| 9 | 0.14 | 0.08 | 0.19 | |
| 12 | 0.14 | 0.09 | 0.10 | |
| 18 | 0.18 | 0.04 | 0.10 | |
| 24 | 0.10 | 0.07 | 0.17 | |

REFERENCES

Karim A.A. and Bhat, Rajeev, "Fish gelatin: properties, challenges, and prospects as an alternative to mammalian gelatins", Food Hydrocolloids 23 (2009) 563-576 Lachmann, L., et al., eds. Lea & Febiger, Philadelphia, PA, pp. 398-412 (1986) Podczeck, Fridrun and Jones, Brian E., Pharmaceutical Capsules, 2004, pp. 195-204 Singh et al., "Microencapsulation: A promising technique for controlled drug delivery", Res Pharm Sci. 2010 Jul-Dec; 5(2): 65-77

CA 2 675 892

WO2012/021432

**Claims**

1. A method for producing a soft gel capsule comprising uncoated probiotic bacteria, the method comprising:

   a) mixing the uncoated probiotic bacteria with at least one oil to obtain a soft gel capsule fill material at a temperature in the range of 5 to 15°C,
   b) encapsulating the fill material in a soft gel capsule made of a gelatin having a melting point in the range of 11 to 28°C; and
   c) drying the soft gel capsule in one or more steps at a temperature of at the most 25°C to a water activity of at the most 0.25,

   wherein during shelf storage the soft gel capsule is dried to an $a_w$ of at the most 0.2 and stored in a resealable container in/on the inside wall(s) of which at least one channel former is embedded, at least over part of the area, together with at least one absorbent.

2. A method according to claim 1 wherein the uncoated probiotic bacteria are non-spore-forming.

3. A method according to claim 1 or claim 2 wherein at least one oil is selected from the group consisting of omega 3s, such as docosahexaenoic acid (DHA), eicosapentaenoic acid (EPA), fish oil, and krill oil.

4. A method according to any one of claims 1 to 3, wherein at least one oil is selected from the group consisting of vegetable oils such as rapeseed oil, borrage oil/evening primrose oil, linseed oil, perilla oil, or garlic oil.

5. A method according to any one of claims 1 to 4 wherein the gelatin has a bloom strength in the range of 100 to 300 bloom.

**6.** A method according to any one of claims 1 to 5 wherein the probiotic bacteria are Lactobacilli, Lactococci, Pediococci, Streptococci or Bifidobacteria, or a mixture thereof.

**7.** A method according to claim 6 wherein the probiotic bacteria are from the strain Bifidobacterium animalis subspecies lactis.

**8.** A method according to any one of claims 1 to 7 wherein the soft gel capsule comprises one, two, three, four or more active ingredients selected from the group consisting of vitamins, such as vitamin A, D, E, K2, C, B2, B6, B12, biotin, niacin, folic acid; minerals, such as zinc, selenium, chromium, copper, calcium, chloride; and vegetable extracts, such as cranberry extract/juice or royal jelly.

**9.** A method according to any one of claims 1 to 8 wherein during shelf storage the soft gel capsule is stored in a container made from polymers which comprise absorbents and channel formers.

**10.** A method according to any one of claim 9 wherein during shelf storage the soft gel capsule is stored in a container made from HDPE polymers or other high water barrier polymers.

**11.** A method according to claim 9 or 10 wherein during shelf storage the soft gel capsule is stored in a container comprising a suitable desiccant such as silica, silica based desiccants, molecular sieves, and zeolites.

**12.** A method according to any one of claims 9 to 11 wherein during shelf storage the soft gel capsule is stored in a plastic vial with desiccant lining.

**13.** A method according to claim 12 wherein during shelf storage the soft gel capsule is stored in a plastic vial with active desiccant lining.

**14.** A soft gel capsule produced by the method according to any one of claims 1 to 13.

**15.** A soft gel capsule according to claim 14 for use as a dietary supplement, nutraceutical or pharmaceutical.

**Patentansprüche**

**1.** Verfahren zur Herstellung einer Weich-Gel-Kapsel, enthaltend unbeschichtete probiotische Bakterien, wobei das Verfahren umfasst:

a) Mischen der unbeschichteten probiotischen Bakterien mit mindestens einem Öl zum Erhalten eines Weich-Gel-Kapsel-Füllmaterials bei einer Temperatur im Bereich von 5 bis 15°C,
b) Einkapseln des Füllmaterials in eine Weich-Gel-Kapsel, die aus einer Gelatine mit einem Schmelzpunkt im Bereich von 11 bis 28°C hergestellt ist; und
c) Trocknen der Weich-Gel-Kapsel in einem oder mehreren Schritten bei einer Temperatur von höchstens 25°C auf eine Wasseraktivität von höchstens 0,25,

wobei die Weich-Gel-Kapsel während Regallagerung auf einen $a_w$ von höchstens 0,2 getrocknet und in einem wiederverschließbaren Behälter gelagert wird, in/auf dessen Innenwand(wänden) mindestens ein Kanalbildner zumindest über einen Teil der Fläche zusammen mit mindestens einem Absorptionsmittel eingebettet ist.

**2.** Verfahren nach Anspruch 1, wobei die unbeschichteten probiotischen Bakterien nicht sporenbildend sind.

**3.** Verfahren nach Anspruch 1 oder Anspruch 2, wobei mindestens ein Öl ausgewählt ist aus der Gruppe, bestehend aus Omega 3 Fettsäuren wie beispielsweise Docosahexaensäure (DHA), Eicosapentaensäure (EPA), Fischöl und Krillöl.

**4.** Verfahren nach einem der Ansprüche 1 bis 3, wobei mindestens ein Öl ausgewählt ist aus der Gruppe, bestehend aus Pflanzenölen wie beispielsweise Rapsöl, Borretschöl/Nachtkerzenöl, Leinsamenöl, Perillaöl oder Knoblauchöl.

**5.** Verfahren nach einem der Ansprüche 1 bis 4, wobei die Gelatine einen Bloom-Wert im Bereich von 100 bis 300 Bloom aufweist.

**6.** Verfahren nach einem der Ansprüche 1 bis 5, wobei die probiotischen Bakterien Lactobacilli, Lactococci, Pediococci, Streptococci oder Bifidobacterien oder deren Mischung sind.

**7.** Verfahren nach Anspruch 6, wobei die probiotischen Bakterien von dem Stamm Bifidobacterium animalis Unterklasse lactis sind.

**8.** Verfahren nach einem der Ansprüche 1 bis 7, wobei die Weich-Gel-Kapsel ein, zwei, drei, vier oder mehr Wirkbestandteile aufweist, die ausgewählt sind aus der Gruppe, bestehend aus Vitaminen wie beispielsweise Vitamin A, D, E, K2, C, B2, B6, B12, Biotin, Niacin, Folsäure; Mineralien wie beispielsweise Zink, Selen, Chrom, Kupfer, Calcium, Chlorid; und Pflanzenextrakte wie beispielsweise Cranberry-Extrakt/-Saft oder Gelee Royale.

**9.** Verfahren nach einem der Ansprüche 1 bis 8, wobei die Weich-Gel-Kapsel während Regallagerung in einem aus Polymeren hergestellten Behälter gelagert wird, der Absorptionsmittel und Kanalbildner enthält.

**10.** Verfahren nach Anspruch 9, wobei die Weich-Gel-Kapsel während Regallagerung in einem Behälter gelagert wird, der aus HDPE-Polymeren oder anderen Polymeren mit hoher Wasserbarriere hergestellt ist.

**11.** Verfahren nach Anspruch 9 oder 10, wobei die Weich-Gel-Kapsel während Regallagerung in einem Behälter gelagert wird, der ein geeignetes Trocknungsmittel wie beispielsweise Kieselsäure, Trocknungsmittel auf Kieselsäurebasis, Molekularsiebe und Zeolithe aufweist.

**12.** Verfahren nach einem der Ansprüche 9 bis 11, wobei die Weich-Gel-Kapsel während Regallagerung in einer Kunststoff-Ampulle mit einer Auskleidung aus Trocknungsmittel gelagert wird.

**13.** Verfahren nach Anspruch 12, wobei die Weich-Gel-Kapsel während Regallagerung in einer Kunststoff-Ampulle mit einer Auskleidung aus aktivem Trocknungsmittel gelagert wird.

**14.** Weich-Gel-Kapsel, hergestellt durch das Verfahren nach einem der Ansprüche 1 bis 13.

**15.** Weich-Gel-Kapsel nach Anspruch 14 zur Verwendung als ein Nahrungsergänzungsmittel, Nutrazeutikum oder Pharmazeutikum.

## Revendications

**1.** Un procédé de production d'une gélule molle comprenant des bactéries probiotiques non enrobées, le procédé comprenant les étapes comprenant:

a) mélanger les bactéries probiotiques non enrobées à au moins une huile pour obtenir un matériau de remplissage de gélule molle à une température dans la plage de 5 à 15°C,
b) encapsuler le matériau de remplissage dans une gélule molle constituée d'une gélatine ayant un point de fusion de 11 à 28 ° C; et
c) sécher la gélule molle en une ou plusieurs étapes à une température d'au plus 25 °C jusqu'à une activité de l'eau d'au plus 0,25,

dans lequel la gélule molle est séchée pendant le stockage sur une étagère jusqu'à un $a_w$ d'au plus 0,2 et stockée dans un récipient pouvant être refermé dont dans / sur la ou les paroi(s) internes au moins un formateur de canaux est intégré, au moins sur une partie de la zone, conjointement avec au moins un absorbant.

**2.** Le procédé selon la revendication 1, dans lequel les bactéries probiotiques non enrobées sont non-sporogènes.

**3.** Le procédé selon la revendication 1 ou la revendication 2, dans lequel au moins une huile est choisie dans le groupe consistant en oméga 3s tels que l'acide docosahexaénoïque (DHA), l'acide eicosapentaénoïque (EPA), l'huile de poisson et l'huile de krill.

**4.** Le procédé selon l'une quelconque des revendications 1 à 3, dans lequel au moins une huile est choisie dans le groupe consistant en huiles végétales telles que l'huile de colza, l'huile de bernage / l'huile d'onagre, l'huile de lin, l'huile de périlla ou l'huile d'ail.

**5.** Le procédé selon l'une quelconque des revendications 1 à 4, dans lequel la gélatine a une valeur de Bloom dans la plage de 100 à 300 bloom.

**6.** Le procédé selon l'une quelconque des revendications 1 à 5, dans lequel les bactéries probiotiques sont des lactobacilles, des lactocoques, des pédiocoques, des streptocoques ou des bifidobactéries ou un mélange de ceux-ci.

**7.** Le procédé selon la revendication 6, dans lequel les bactéries probiotiques proviennent de la souche Bifidobacterium animalis sous-espèce lactis.

**8.** Le procédé selon l'une quelconque des revendications 1 à 7, dans lequel la gélule molle comprend un, deux, trois, quatre ou plus de principes actifs choisis dans le groupe consistant en vitamines, telles que les vitamines A, D, E, K2, C, B2, B6, B12, biotine, niacine, acide folique; des minéraux, tels que le zinc, le sélénium, le chrome, le cuivre, le calcium, le chlorure; et des extraits végétaux tels que l'extrait / le jus de canneberge ou la gelée royale.

**9.** Le procédé selon l'une quelconque des revendications 1 à 8, dans lequel pendant le stockage sur étagère la gélule molle est stockée dans un récipient fabriqué par des polymères comprenant des absorbants et des formateurs des canaux.

**10.** Le procédé selon la revendication 9, dans lequel pendant le stockage sur étagère la gélule molle est stockée dans un récipient fabriqué par des polymères d'HDPE ou des polymères pare-eau.

**11.** Le procédé selon la revendication 9 ou 10, dans lequel pendant le stockage sur étagère la gélule molle est stockée dans un récipient comprenant un déshydratant approprié tel que la silice, des déshydratants à base de silice, des tamis moléculaires et des zéolites.

**12.** Le procédé selon l'une quelconque des revendications 9 à 11, dans lequel pendant le stockage sur étagère la gélule molle est stockée dans une fiole en plastique avec un revêtement de déshydratant.

**13.** Le procédé selon la revendication 12, dans lequel pendant le stockage sur étagère la gélule molle est stockée dans une fiole en plastique avec un revêtement de déshydratant actif.

**14.** Une gélule molle, produite par le procédé selon l'une quelconque des revendications 1 à 13.

**15.** La gélule molle selon la revendication 14, destinée à être utilisée comme un complément alimentaire, un produit nutraceutique ou un produit pharmaceutique.

Figure 1

Figure 2

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012021432 A
- CA 2675892
- US 2012107395 A
- WO 2009095670 A
- WO 9732663 A
- WO 03086900 A
- WO 2008086852 A
- EP 1000873 A
- EP 1421991 A

### Non-patent literature cited in the description

- Part Two. Soft Gelatin Capsules. **STANLEY, J.P. et al.** The Theory and Practice of Industrial Pharmacy. Lea & Febiger, 1986, 398-412
- Fish gelatin: properties, challenges, and prospects as an alternative to mammalian gelatins. **KARIM A.A. ; BHAT, RAJEEV.** Food Hydrocolloids. 2009, vol. 23, 563-576
- **PODCZECK, FRIDRUN ; JONES, BRIAN E.** Pharmaceutical Capsules. 2004, 195-204
- **SINGH et al.** Microencapsulation: A promising technique for controlled drug delivery. *Res Pharm Sci.,* July 2010, vol. 5 (2), 65-77